# EUROPEAN PATENT SPECIFICATION

(11) **EP 2 117 453 B1**
(45) Date of publication and mention of the grant of the patent: **24.10.2012**
(21) Application number: 07865248.4
(22) Date of filing: 05.12.2007
(51) Int. Cl.: A61B 17/82, A61B 17/88

(54) **CRIMP PLIERS**
KRÄUSELZANGE
PINCES À SERTIR

(30) Priority: 12.12.2006 US 874261 P
(43) Date of publication of application: 18.11.2009
(73) Proprietor: Synthes GmbH, 4436 Oberdorf (CH)
(72) Inventor: DELL'OCA, Alberto A. Fernandez, Montevideo (UY)
(74) Representative: Lusuardi, Werther
(86) International application number: PCT/US2007/086523
(87) International publication number: WO 2008/073782

(56) References cited:
- WO-A-2007/124100
- US-A- 4 587 963
- US-A- 4 966 600
- US-A1- 2002 095 153
- US-B1- 6 383 200

## Description

### Field of the Invention

The present invention relates to orthopedics, and more particularly to devices for securing surgical cable around bone.

### Background

Bone cerclage in which a cable is looped about a fracture site and tightened in place using a crimp is a well known technique. A wire passer as described in U.S. Patent Application Serial No. 11/194,642 to A.Femández Dell'Oca filed August 2, 2005 and Published February 22, 2007 as 20070043377 ("the '642 application"), allows the insertion and loop of the cable around the fractured bone to be achieved via a small incision, minimizing trauma and discomfort.
From US 4,966,600 SONGER a device for securing a cable around a bone is known which forms the basis for the preamble of claim 1.

However, the space required to operate existing pliers for deforming the crimp causes spreading of this incision and significant muscle trauma.

### Summary of the Invention

The present invention provides an osteosynthetic device permitting minimally invasive crimping of a cable looped around a fractured bone and which reduces the time required for and tissue damage associated with the procedure.

Apparatus according to one embodiment of the present invention includes a pair of crimp pliers comprising a pair of handles, two connected opposed jaws that reach the deeply located crimp through a minimal incision, two guiding ventral protrusions designed to guide the introduction of the pliers along an intermediate part of the cable, a depth level which indicates to the operator when the jaws are in position over the crimp to crush the deeply located crimp next to the fractured bone. The pliers include a lock which is released at this point so that the handles may be pulled together forcing the distal ventral protrusions to close over and deform the crimp.

### Brief Description of the Drawings

Fig. 1 is a perspective view of the fractured bone with a cable looped therearound connected to a crimp for use in conjunction with the present invention;
Fig. 2 is a perspective view of a crimp pliers according to the invention running along a tube through which the cable extends to the crimp;
Fig 3 is a perspective view of the crimp pliers positioned over the crimp;
Fig 4 is a perspective view of the crimp pliers with a lock released; and
Fig 5 is a perspective view of the pliers in an actuated position crushing the crimp.
Fig. 6 is a perspective view of a bone cerclage tool that may be used to pass a wire around a fractured bone wherein the two separate members are not coupled.
Fig. 7 is a side view of the bone cerclage tool of Fig. 6 inserted about a bone and wherein the two members are coupled together.
Fig. 8 is a side view of the bone cerclage tool of Fig. 6, wherein the tool is disassembled to be removed from a body.

### Detailed Description

The present invention, which may be further understood with reference to the following description and appended drawings, wherein like elements are provided with the same reference numerals. The present invention relates to devices for treating fractures and, more specifically, relates to a device for minimally invasively securing cable around a fractured bone. Exemplary embodiments of the present invention provide a means for minimally invasively compressing a crimp over surgical cable looped around a fractured bone to maintain tension within the cable and stabilize the fracture.

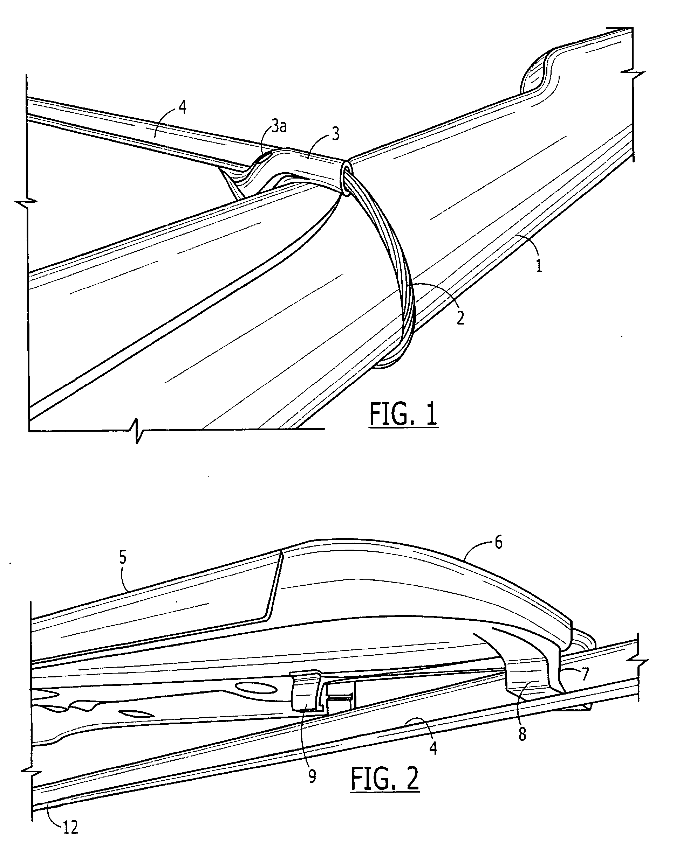

As shown in Figs. 1 - 5, a system according to an exemplary embodiment of the present invention includes a crimp pliers 5 for compressing a crimp 3 over a cable 2 looped around a fractured bone 1 to lock the cable 2 in position and maintain the portions of the fractured bone 1 in a desired spatial relation to one another. The crimp pliers 5 includes a pair of operating handles 13a and 13b, opposed jaws 6a and 6b, a pair of distal crimp engaging protrusions 8 thereof and a pair of proximal protrusions 9. The crimp pliers 5 according to this embodiment further comprises a locking mechanism 10 and a depth level 11. As will be described in more detail below, the crimp pliers 5 is guided to the crimp 3 by sliding the proximal protrusions 9 along a tube 4 through which the cable 2 extends to the crimp 3. Once the opposed jaws 6a, 6b have reached the crimp 3, the handles 13a, 13b are drawn toward each other to bring the distal protrusions 8 together over the crimp 3 to crush the crimp 3 over the cable 2.

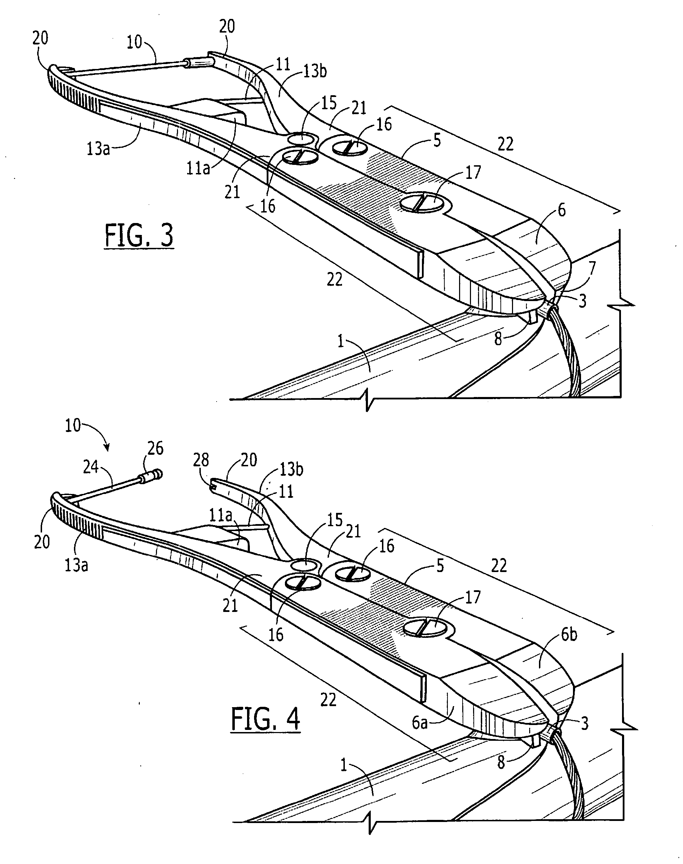

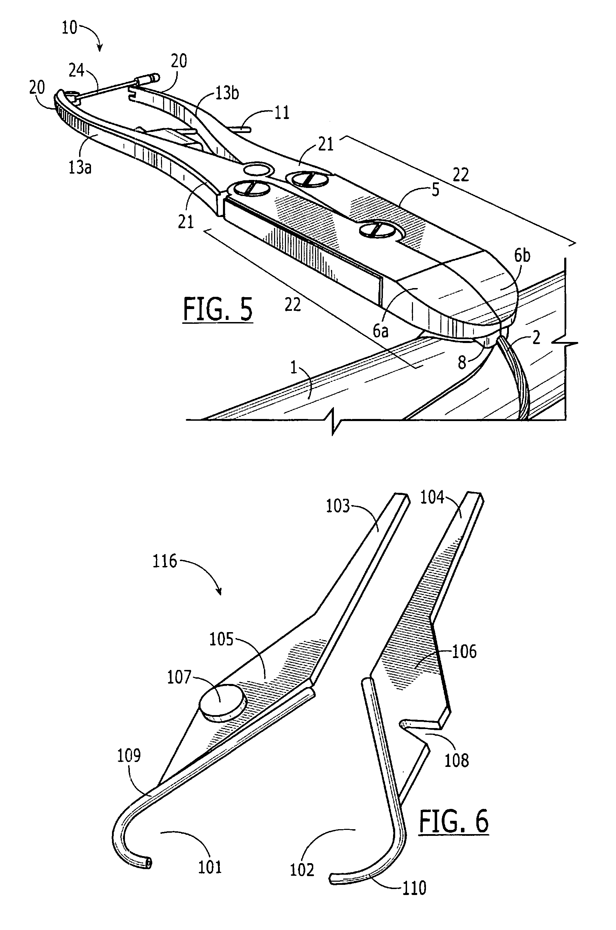

The operating handles 13a, 13b and the opposed jaws 6a, 6b are connected such that drawing the operating handles 13a, 13b toward one another brings the opposed jaws 6a, 6b toward one another applying a compressive force to anything located between the jaws 6a, 6b. Furthermore, pulling the operating handles 13a, 13b away from one another opens the opposed jaws 6a, 6b. For example, in this embodiment, the operating handles 13a, 13b are rotatably coupled to one another at a pivot 15 so that moving the proximal ends 20 toward one another as shown in Fig. 5, moves distal ends 21 thereof away from one another. Each of the distal ends 21 of the handles 13a, 13b is pivotally coupled to a corresponding distal lever 22 at a pivot 16 and the levers 22 are rotatably coupled to one another at a pivot 17 so that, as the distal ends 21 of the handles 13a, 13b draw the proximal ends of the levers 22 away from one another, the jaws 6a, 6b at the distal ends of the levers 22 are rotated toward one another. When the handles 13a, 13b are moved away from one another, this process is reversed and the proximal ends of the levers 22 are drawn toward one another separating the jaws 6a, 6b from one another as shown in Figs. 3 and 4.

At a distal tip 7 of the opposed jaws 6a, 6b are the distal protrusions 8, which extend ventrally from the opposed jaws 6a, 6b such that one protrusion extends from each jaw 6a, 6b. The distal protrusions 8 are formed such that when the opposed jaws 6a, 6b are drawn closed the distal protrusions 8 come together to compress the crimp 3. The proximal protrusions 9 also extend ventrally from the crimp pliers 5 and may be formed proximal to the distal protrusions 8. The proximal protrusions 9 are sized and shaped accommodate the tube 4 between them in order to slide along the tube 4 to guide the crimp pliers 5. The crimp pliers 5 is guided such that the distal tip 7 of the opposed jaws 6a, 6b and, consequently, the distal protrusions 8, are positioned over the crimp 3.

As indicated above, a locking mechanism 10 operates to prevent movement of the handles 13a, 13b relative to one another as the pliers 5 is moved into position over the crimp 3. The locking mechanism according to this embodiment includes a bar 24 one end of which is pivotally coupled to the handle 13a while a second end includes a dowel 26 which, when in a locked position, is received within a slot 28 of the handle 13b. Thus, when engaged with the handle 13b, the bar 24 maintains a separation between the handles 13a, 13b, which corresponds to a maximum separation of the jaws 6a, 6b. When it is desired to move the handles 13a, 13b relative to one another (e.g., when the crimp 3 is positioned between the distal protrusions 8), the locking mechanism 10 is released by rotating the bar 24 to remove the dowel 26 from the slot 28. At this point the handles 13a, 13b are free to move relative to one another.

A depth level 11 is also formed between the operating handles 13a, 13b of the pliers 5 according to this embodiment of the invention. However, those skilled in the art will understand that the pliers 5 may be formed without either or both of the depth level 11 and the locking mechanism 10. These components are optional. The depth level 11 according to this embodiment, comprises a shaft that is attached to only one of the operating handles 13a, 13b (in this case, 13a) such that the operating handles 13a, 13b may still be drawn together without interference from the depth level 11. The depth level 11 includes a distally facing abutting surface 11a located at a predetermined distance from the distal tip 7 of the distal protrusions 8.

As shown in Fig. 1, a flexible cable 2 is looped around the fractured bone 1. It will be understood by those in the art that the cable 2 may be looped around the bone 1 using any known technique. For example, the cable 2 may be looped around the bone 1 using a minimally invasive wire passing means such as that disclosed in U.S. Patent Application Publication No. 2007/0043377 by Alberto Fernandez Dell' Oca As shown in Figs. 6 - 8, such a cerclage tool comprises two members, each having a handle, a central part and a J shaped tube. When the central part of both members is firmly coupled together, both J shaped tubes conform a continuous tube through which a wire, cable, band or suture can be fed.

As shown in Fig. 6, the wire/cable passer 116 has two members 101 and 102, each including a handle 103, 104 by which the cable passer 116 is gripped and to bring members 101, 102 together when the handles 103, 104 are pulled close to one another. The member 101 includes a button 107 on a central part 105 thereof, while the member 102 includes a notch 8 in a central part 106 thereof. When the members 101, 102 are drawn together, J shaped tubes 109, 110 form a continuous tube through which a wire 111 cable, band or suture can be fed.

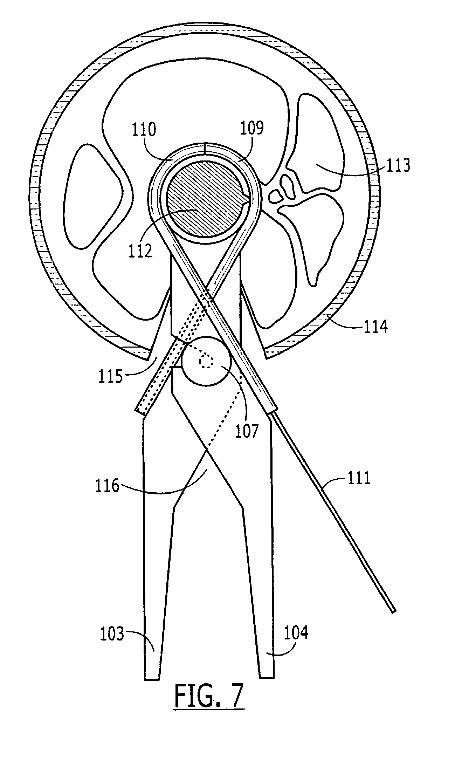

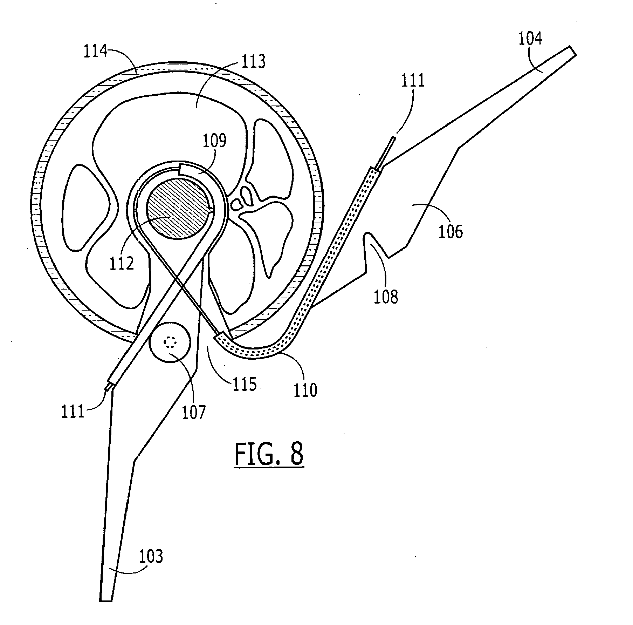

In use, the members 101, 102 are successively inserted close to a bone 112 through a small skin incision 115, minimally disturbing skin 114 and underlying muscle 113, as shown in Fig. 7. After both members 101, 102 have been inserted, they are drawn firmly together by pulling handles 103, 104 until the button 107 is inserted into the notch 108. The wire 111 can then be fed through the continuous tube formed by the two J shaped tubes 109, 110 which now surround the bone 112. The tool 16 is then disassembled and removed, as shown in Fig. 8 leaving the wire 111 in place looped about the bone 112.

Generally a cable 2 with one ball end and one free end is used. The free end of the cable 2 is inserted into a first one of the lumens of a crimp 3 having 2 side-by-side lumens and the free end of the cable 2 is pulled through the crimp 3 until the ball end lodges in the end of the crimp 3. The free end of the cable 2 is then passed around the bone 1, drawn out through the incision and inserted into the second lumen of the crimp 3 via an opening 3a. The crimp 3 is slid down the cable 2 until it reaches the bone 1 and the free end of the cable 2 is passed through a tube 4 which is slid over the cable 2 until a distal end of the tube 4 abuts the crimp 3 adjacent to the opening 3a, as shown in Fig. 1. A distal end of the tube 4 is shaped to conform to a shape of a proximal side of the crimp 3 so that it fits snugly thereagainst. In this position, a distal end of the tube 4 and the proximal side of the crimp 3 are aligned with one another to form a substantially continuous outer surface. After the cable 2 has been passed through the crimp 3 and the tube 4, a tensioning device is attached to the end of the cable 2 extending from the tube 4 and tensioned as would be understood by those skilled in the art until a desired tension is obtained to maintain the fragments of the fractured bone 1 in a desired spatial relationship to one another.

Once the fracture has been reduced and the cable 2 is at the desired tension, crimp pliers 5 may be used to crush the crimp 3 over the cable to maintain the desired tension in the cable 2. The distal protrusions 8 are placed on opposite sides of the tube 4 with the jaws 6a, 6b in the open state and the proximal protrusions 9 also engage the tube 4, as shown in Fig. 2. The crimp pliers 5 is then slid along the tube 4 until a proximal end 12 of the tube 4 abuts the depth level 11, as shown in Fig. 3. The length of the tube 4 is selected so that, when the proximal end 12 abuts the proximal face 11a of the depth level 11, the distal protrusions 8 are located on opposite sides of the crimp 3.

As shown in Fig. 4, after it is determined that the crimp pliers 5 is appropriately positioned, the lock mechanism 10 is released to free the handles 13a, 13b for movement toward one another. The user then draws the handles 13a, 13b together closing the jaws 6a, 6b and drawing the protrusions 8 toward one another crushing the crimp 3 over the cable 2. As shown in Fig. 5, since the level 11 and the lock 10 are only attached to one of the operating handles 13a, the level 10 and lock 11 do not interfere with the drawing closed of operating handles 13a, 13b. Once the crimp 3 has been compressed and deformed over the cable 2, the cable 2 maintains the desired tension and holds the fractured pieces of the bone 1 together in the desired spatial relationship. The tube 4 is then removed and the portion of the cable 2 extending from the opening 3a is cut and removed from the body. The crimp pliers 5 disclosed herein allows the desired tension to be maintained on the cable 2 without requiring significant spreading of the incision, reducing muscle trauma and tissue damage.

It will be apparent to those skilled in the art that various modifications and variations can be made in the structure of the present invention, without departing from the scope of the invention. Thus, it is intended that the present invention cover the modification and variations of this invention provided that they come within the scope of the appended claims and their equivalents.

## Claims

1. A device for securing cable around a bone, comprising:
first and second handles (13a; 13b) pivotally coupled to one another;
a first distal member (6a) a proximal end of which is pivotally coupled to a distal end (21) of the first handle (13a), the first distal member (6a) including a first crimp engaging protrusion (8) extending away from a ventral surface thereof at a distal end thereof;
a second distal member (6b) a proximal end of which is pivotally coupled to a distal end (21) of the second handle (13b), the second distal member (6b) including a second crimp engaging protrusion (8) extending away from a ventral surface thereof at a distal end thereof; and
a depth level (11) including an abutting surface (11a),
the first and second distal members being pivotally coupled to one another so that, when the first and second handles (13a; 13b) are drawn toward one another, the proximal ends of the first and second distal members (6a; 6b) are moved apart from one another, drawing the first and second protrusions (8) toward one another to compress a crimp located therebetween
**characterized in that**
the device comprises a depth measuring tube (4) adapted to extend over a portion of a cable extending proximally from a crimp, wherein
contact between the abutting surface (11a) and the depth measuring tube (4) indicating to a user that the first and second protrusions are located on opposite sides of the crimp.

2. The device of claim 1, further comprising a lock mechanism (10) which, when engaged, prevents relative movement between the first and second protrusions (8).

3. The device of claim 2, wherein the lock mechanism (10) includes a bar (24) pivotally connected to one of the first and second handles (13a;13b) and releasably coupled to the other of the first and second handles (13a;13b).

4. The device of claim 3, wherein the bar (24) of the lock mechanism (10) includes a dowel (26) at an end thereof which, when in a locked position, is received within a slot (28) of the other one of the first and second handles (13a;13b).

## Patentansprüche

1. Vorrichtung zum Sichern eines Kabels um einen Knochen, umfassend:
erste und zweite Handgriffe (13a, 13 b), welche rotierbar miteinander verbunden sind;
ein erstes distales Glied (6a) mit einem rotierbar mit einem distalen Ende (21) des ersten Handgriffs (13a) verbundenen proximalen Ende, wobei das erste distale Glied (6a) einen ersten mit einem Klemmverbinder in Eingriff bringbaren Vorsprung (8) umfasst, welcher sich von einer ventralen Fläche davon und an einem distalen Ende davon ausdehnt;
ein zweites distales Glied (6b) mit einem rotierbar mit einem distalen Ende (21) des zweiten Handgriffs (13b) verbundenen proximalen Ende, wobei das zweite distale Glied (6a) einen zweiten mit einem Klemmverbinder in Eingriff bringbaren Vorsprung (8) umfasst, welcher sich von einer ventralen Fläche davon und an einem distalen Ende davon ausdehnt; und
eine Tiefenanzeige (11) mit einer Stirnfläche (11a), wobei
das erste und zweite distale Glied rotierbar miteinander verbunden sind, so dass, wenn die ersten und zweiten Handgriffe (13a, 13b) gegeneinander gezogen werden, die proximalen Enden der ersten und zweiten distalen Glieder (6a, 6 b) voneinander weg bewegt werden, wobei der erste und zweite Vorsprung (8) gegeneinander gezogen werden, um einen dazwischen angeordneten Klemmverbinder zu komprimieren, **dadurch gekennzeichnet, dass**
die Vorrichtung ein Tiefenmessrohr (4) umfasst, welches so angepasst ist, dass es sich über einen Teil eines Kabels erstreckt, welches sich proximal von einem Klemmverbinder ausdehnt, wobei
ein Kontakt zwischen der Stirnfläche (11a) und dem Tiefenmessrohr (4) einem Benutzer anzeigt, dass der erste und zweite Vorsprung auf gegenüberliegenden Seiten des Klemmverbinders angeordnet sind.

2. Vorrichtung nach Anspruch 1, zusätzlich umfassend einen Sperrmechanismus (10), welcher, wenn er im Eingriff ist, eine relative Bewegung zwischen dem ersten und zweiten Vorsprung (8) verhindert.

3. Vorrichtung nach Anspruch 2, **dadurch gekennzeichnet, dass** der Sperrmechanismus (10) einen Stab (24) umfasst, welcher rotierbar mit einem der ersten und zweiten Handgriffe (13a, 13b) verbunden ist und lösbar mit dem anderen der ersten und zweiten Handgriffe (13a, 13b) verbunden ist.

4. Vorrichtung nach Anspruch 3, **dadurch gekennzeichnet, dass** der Stab (24) des Sperrmechanismus (10) an einem seiner Enden einen Passstift (26) umfasst, welcher, wenn in einer gesperrten Position, in einem Schlitz (28) des anderen der ersten und zweiten Handgriffe (13a, 13 b) aufgenommen ist.

## Revendications

1. Dispositif de fixation d'un câble autour d'un os, comprenant :
des premier et second manches (13a ; 13b) couplés de façon pivotante l'un à l'autre ;
un premier organe distal (6a) dont une extrémité proximale est couplée de façon pivotante à une extrémité distale (21) du premier manche (13a), le premier organe distal (6a) comprenant une première protubérance de mise en prise de sertissage (8) s'étendant en éloignement d'une surface ventrale de ce dernier au niveau d'une extrémité distale de ce dernier ;
un second organe distal (6b) dont une extrémité proximale est couplée de façon pivotante à une extrémité distale (21) du second manche (13b), le second organe distal (6b) comprenant une seconde protubérance de mise en prise de sertissage (8) s'étendant en éloignement d'une surface ventrale de ce dernier au niveau d'une extrémité distale de ce dernier ; et
un niveau de profondeur (11) comprenant une surface de butée (11a),
les premier et second organes distaux étant couplés de façon pivotante l'un à l'autre de sorte que, lorsque les premier et second manches (13a ; 13b) sont rapprochés l'un de l'autre, les extrémités proximales des premier et second organes distaux (6a ; 6b) sont éloignées l'une de l'autre, rapprochant les première et seconde protubérances (8) l'une de l'autre afin de comprimer un sertissage situé entre ces dernières,
**caractérisé en ce que**
le dispositif comprend un tube de mesure de profondeur (4) adapté pour s'étendre sur une portion d'un câble s'étendant de façon proximale depuis un sertissage, dans lequel
un contact entre la surface de butée (11a) et le tube de mesure de profondeur (4) indique à un utilisateur que les première et seconde protubérances sont situées à des côtés opposés du sertissage.

2. Dispositif selon la revendication 1, comprenant en outre un mécanisme de verrou (10), qui lorsqu'il est mis en prise, empêche un mouvement relatif entre les première et seconde protubérances (8).

3. Dispositif selon la revendication 2, dans lequel le mécanisme de verrouillage (10) comprend une barre (24) raccordé de façon pivotante à l'un des premier et second manches (13a ; 13b) et couplée de façon amovible à l'autre des premier et second manches (13a ; 13b).

4. Dispositif selon la revendication 3, dans lequel la barre (24) du mécanisme de verrouillage (10) comprend un goujon (26) à une de ses extrémités qui, en position verrouillée, est reçu à l'intérieur d'une fente (28) de l'autre des premier et second manches (13a ; 13b).
